# EUROPEAN PATENT SPECIFICATION

(11) **EP 3 915 516 B1**
(45) Date of publication and mention of the grant of the patent: **11.02.2026**
(21) Application number: 20826661.9
(22) Date of filing: 24.02.2020
(51) Int. Cl.: A61B 17/00, A61B 17/06

(54) **LIFTING DEVICE**
LIFTING VORRICHTUNG
DISPOSITIF DE LIFTING

(30) Priority: 19.06.2019 CN 201910531045
(43) Date of publication of application: 01.12.2021
(73) Proprietor: Tongyan (Shanghai) Medical Device Co., Ltd., Shanghai 201203 (CN)
(72) Inventor: YAN, Wei, Shanghai 201203 (CN); XIA, Peipei, Shanghai 201203 (CN); WEI, Zheng, Shanghai 201203 (CN)
(74) Representative: HGF
(86) International application number: PCT/CN2020/076357
(87) International publication number: WO 2020/253256

(56) References cited:
- EP-A1- 3 225 175
- CN-A- 103 251 433
- CN-A- 103 251 433
- CN-A- 103 889 340
- CN-A- 109 864 835
- CN-A- 110 123 485
- CN-U- 207 804 289
- CN-U- 210 521 145
- CN-Y- 201 404 310
- GB-A- 2 535 799
- GB-A- 2 540 293
- KR-A- 20190 059 673
- US-A1- 2010 160 961
- US-A1- 2017 281 160

## Description

### TECHNICAL FIELD

The present application pertains to the technical field of medical equipment and relates to a one-way lifting embedded thread, a lifting device, and a plastic surgery method using the same.

### BACKGROUND

Factors including age and environment may lead to the soft tissue and bone volume of a human face shrink, ligaments start to loosen, and the superficial musculoaponeurotic system gradually droop. In the combined action of gravity and flabby zygomatic buccal fat pad, the superficial tissue of the face becomes sagging, with elasticity decreasing, wrinkles increasing, and grooves forming and deepening. Moreover, along with aging, factors including changes in the hormone level inside the body, losses in collagen, and the atrophy in facial glands co-work to result in an adverse physical change in the face, that is, aging.

With the progress and development of society, people gradually heighten their demands for beauty, especially demands for facial rejuvenation. Moreover, the demands tend to grow year by year. Currently, to improve and maintain facial rejuvenation, a specially-made thread made of an absorbable biomaterial is introduced into the shallow soft tissue through a minimally invasive operation. The loose facial soft tissue is lifted and restored through the excellent lifting of the thread and the action of mechanically equal distribution.

Targeted lifting may be carried out on different parts of the face and directly act on the flabby skin. Additionally, the material for manufacturing the thread is a biodegradable and absorbable material with good biocompatibility. After a period of time, the thread can not only be degraded and absorbed by the facial tissue, but also stimulate the regeneration of collagen in the subcutaneous tissue, helping form new supporting ligaments and elastic fibers, thickening the facial skin, and preventing skin aging.

CN207804289U discloses a new-type cosmetic lifting thread. The cosmetic lifting thread includes a pulling thread and needles connected to two ends of the pulling thread. Two groups of lifting members are disposed symmetrically on the pulling thread. The lifting members are trumpet-shaped. The pulling thread strings the two groups of lifting members. The two groups of lifting members are disposed symmetrically on two sides of the middle section of the pulling thread. Each small head of each group of lifting members faces the needle of the pulling thread on the same side. A non-slip knot is disposed inside the small head of the lifting member. The size of the non-slip knot is more than the internal bore diameter of the small head.

The related lifting and wrinkle removing products in the market are various and of complete functions. However, most of them are two-way lifting embedded threads that need to be inserted into visible parts of the face, implanted symmetrically, and then lifted. In this case, a wound is formed, affecting the facial beauty of the patient.

CN103251433A discloses a suture assembly, the suture assembly includes an elongated flexible body which may be either a single filament for a multiple strand braided or woven body, supporting a plurality of shaped tissue-engaging elements in a generally spaced arrangement thereon. A curved body having a sharp point is joined to one end of the flexible body while a straight pointed body is joined to the remaining end of the flexible body. In alternate embodiments, the tissue-engaging elements define a variety of cross-section shapes. In a further alternate embodiment, the tissue-engaging elements are divided into first and second oppositely facing sets to provide a bidirectional suture. In a still further alternate embodiment, the elongated flexible body supports an elongated tissue-bonding sleeve.

CN109864835A discloses a suture line, a suture device and application of the suture line. The suture line includes the following parts: an elongated body including a first free end and a second free end; a first cam assembly and a second cam assembly arranged on the elongated body in a sleeving manner, and each of the first cam assembly and the second cam assembly comprises at least one cam; the cam comprises a small end and a large end, the small end direction of the cam of the first cam assembly faces the first free end direction of the elongated body, and the small end direction of the camof the second cam assembly faces the second free end direction of the elongated body; the sectional area of the small end of the cam is smaller than that of the large end of the cam; the wall thickness of the cam is gradually reduced from the small end to the large end; the cam can be stably fixed to the suture line through the suture line, the phenomenon that the cam and the silk thread relatively slide in the process that the suture line punctures into tissue and the cam begins to be meshed with the tissue is prevented, and therefore the problems that the cam deviates and stress is uneven are solved.

KR20190059673A discloses a surgical instruction for skin lifting, which forms a flat rotation prevention unit to properly prevent the rotation and position change of a lifting chamber, and allows the lifting chamber to be properly arranged in a correct position. The surgical instruction for skin lifting of the present invention comprises: a needle formed of a circular tube of a metal material, and having an insertion hole formed by cutting a part of an upper surface of a front end thereof, and having a part thereof to be vertically pressed to flatten a circular hole to be an oval shape so as to form a first rotation prevention unit; a handle mounted at a rear end of the needle, and having a horizontal communication hole to communicate with the needle; and a lifting chamber inserted into the needle through the insertion hole, provided inside the needle in a state that rotation is prevented by the first rotation prevention unit, formed with a plurality of a barb-shaped protrusions at the outer circumference thereof, and formed in a planar structure.

CN 207804289U discloses a novel cosmetic carrying is acted as go -between, is including the syringe needle of acting as go -between and both ends are connected, the last symmetry of acting as go -between sets up two sets of promotions piece, promotes the piece and is tubaeform, acting as go -between and go here and there two sets of promotions, two sets of promotion symmetric distribution is inthe both sides of acting as go -between the middle section, and the equal syringe needle of acting as go -between of orientation rather than the homonymy of the microcephaly that promotes of every group, and a microcephaly inboard that promotes sets up anti -skidding the knot, and the anti -skidding size of tying is greater than capitular bore diameter. The utility model discloses can solve priorart carry act as go -between easily disconnected, the degradation time short, the newborn defect of unable skin irritation collagen.

GB 2540293A discloses a bio absorbable suture 1 made of poly lactic acid (PLA) having a plurality of beads along its length, the beads also made of poly lactic acid. The beads are preferably a mix of cone shaped beads 2 and substantially spherical or ball shaped beads 3, the cone shaped beads arranged so that some face in each direction along the length of the suture. Preferably a pair of beads comprising one substantially spherical bead and one conical bead are positioned between knots 4 in the suture to limit the movement of the beads along the suture. The suture is suitable for use during cosmetic surgery procedures including face lifts.

EP 3225175A1 discloses a surgical suture (100) is provided, which includes a hollow cone (10, 40, 50) and a thread (20). The cone (10, 40, 50) has a front opening (12, 42, 52) and a rear opening (14, 44, 54) at two opposite ends thereof, wherein a diameter (D1) of the front opening (12, 42, 52) is smaller than a diameter (D2) of the rear opening (14, 44, 54). The thread (20) passes through the cone (10, 40, 50), and integrally includes at least two protrusions (22) thereon, which are separated from each other by a certain distance. Each of the protrusions (22) has a width (W) in a radial direction of the thread (20), wherein the width (W) is greater than a diameter (D) of the thread (20), and is smaller than the diameter (D2) of the rear opening (14, 44, 54). Whereby, with the integrally provided protrusions (22), the cones (10, 40, 50) can be located on the corresponding positions of the thread (20).

GB 2535799A discloses a suture (100) for cosmetic surgery comprises a bioresorbable elongate filament structure (102) having first and second ends (104, 106). At least ten bioresorbable frusto-conical elements (108) suitable for subcutaneous tissue engagement are received movably upon the filament structure. At least twelve protrusions (110) are disposed along the filament structure wherein the elements and protrusions are disposed as spatially-separated first and second sets (116, 118), each of at least 5 elements and at least 6 protrusions. The first set is proximal to the first needle and distal to the second needle, and the second set is proximal to the second needle and distal to the first needle. The elements are orientated in a bidirectional manner such that, when the filament structure is taut, the narrower ends of the elements in the first and second sets are orientated towards the first and second needles. When said elements are in the furthest position from their respective proximal needles that is allowed by the configuration of the suture (120, 122), and the points at which the filament structure is coupled to said needles (124, 126), is at least 10 cm (128, 130).

### SUMMARY

According to disadvantages in the related art, an object of the present application is to provide a one-way lifting embedded thread, a lifting device, and a plastic surgery method using the same. The features of the device according to the present invention are defined in the independent claim.

Further improvements are provided in the dependent claims. The one-way lifting embedded thread has advantages including large lifting force, long lifting duration, simple surgery operation, and no apparent surgical sign at a visibly facial part after the surgery. To achieve this object, the present application adopts the technical solutions below.

In a first aspect, the present application provides a one-way lifting embedded thread. The one-way lifting embedded thread includes a pulling thread and at least one cam body sleeved on the pulling thread.

Two ends of the pulling thread are a lifting end and a free end, respectively. The free end is provided with an anchor structure for securing the pulling thread and preventing the pulling thread from moving toward the lifting end. The free end is configured to stay outside a skin tissue through the anchor structure and adjust a lifting force of the pulling thread after the pulling thread is threaded into the skin issue.

The lifting end is configured to stay inside the skin tissue after the free end is secured by the anchor structure for one-way lifting;

Two sides of the cam body are a large end face and a small end face, respectively. The large end face of the cam body faces the free end of the pulling thread.

The anchor structure comprises a hollow knot or a clamp structure, and the clamp structure comprises a one-way barb structure disposed on the pulling thread and a clamp head interacting with the barb structure to implement one-way locking.

The clamp structure is configured to be capable of moving only towards the free end after the clamp head and the barb structure are locked.

The lifting end and the free end are terms created by inventors. The lifting end refers to the end that is at the pulling thread and firstly enters the skin tissue after the one-way lifting embedded thread is pricked into the skin tissue. The pulling end helps lift the skin tissue and remove wrinkles on the skin tissue. The free end refers to the end staying outside the skin tissue after the one-way lifting embedded thread is pricked into the skin tissue. After the surgery, the anchor structure of the free end is configured to secure with the skin tissue and prevent the one-way lifting embedded thread from slipping into the tissue.

The large end face and the small end face are distinguished by the magnitude of the cross-sectional area. A cam body used in the present application is a non-equal-diameter column structure. The end with a smaller cross-sectional area is defined as the small end face; the end with a larger cross-sectional area is defined as the large end face.

The present application improves the related two-way lifting mode into the one-way lifting mode by providing an anchor structure, thus preventing the lifting embedded thread from completely slipping into the skin tissue, enhancing the lifting force, and shortening lifting operation duration.

In an embodiment, 4 to 24 cam bodies are sleeved on the pulling thread. The number of cam bodies, for example, may be 4, 5, 6, 7, 8, 9, 10, 11, 12, 13, 14, 15, 16, 17, 18, 19, 20, 21, 22, 23, or 24.

In an embodiment, the cam bodies are equally spaced apart on the pulling thread.

In an embodiment, the distance between two adjacent cam bodies is in the range of 2.5 mm to 15 mm, for example, 2.5 mm, 3 mm, 3.5 mm, 4 mm, 4.5 mm, 5 mm, 5.5 mm, 6 mm, 6.5 mm, 7 mm, 7.5 mm, 8 mm, 8.5 mm, 9 mm, 9.5 mm, 10 mm, 10.5 mm, 11 mm, 11.5 mm, 12 mm, 12.5 mm, 13 mm, 13.5 mm, 14 mm, 14.5 mm, or 15 mm. Further in an embodiment, the distance between two adjacent cam bodies is 5.5 mm.

The number of cam bodies and the distance between adjacent cam bodies have a certain matching relation. Those skilled in the art need to make appropriate adjustments according to the physical condition of a patient and the plastic lifting requirement of the patient.

In an embodiment, the cam bodies are unequally spaced apart in gradient on the pulling thread. Those skilled in the art need to select an appropriate gradient distribution law according to the physical condition of the patient and the plastic lifting requirements of the patient.

In an embodiment, the anchor structure is a hollow knot or a clamp. The anchor structure is configured to clamp the one-way lifting embedded thread and prevent the one-way lifting embedded thread from completely slipping into the skin tissue and causing a surgery failure. Additionally, a related lifting embedded thread is mostly in the two-way lifting operation mode, which has two problems. On one hand, a symmetrical position of the face needs to be pricked so that a needle-stab wound is undoubtedly formed at a visibly facial position, resulting in a surgical sign after the surgery and affecting the facial beauty of the patient. On the other hand, part of the cam bodies in the two-way lifting embedded thread are configured to clamp the skin tissue, while the rest of the cam bodies are configured to implement lifting. The number of cam bodies is closely related to the lifting force and the lifting duration. The larger number of cam bodies indicates a larger lifting force and the longer lifting duration. In this manner, under the premise of the same number of cam bodies, the lifting effect of two-way lifting is apparently worse than the lifting effect of one-way lifting. Accordingly, the present application improves the structure of the two-way lifting embedded thread and adds an anchor structure to the free end of the pulling thread. The anchor structure replaces part of the cam bodies in the two-way lifting embedded thread that are configured to clamp the skin tissue, thus preventing the lifting embedded thread from completely slipping into the skin tissue, enhancing the lifting force, and shortening lifting operation duration.

It is to be noted that the hollow knot and the clamp are not knotting modes or conventional members that are well known to those skilled in the art. The hollow knot and the clamp are specifically described below.

In the present application, the knotting mode of the hollow knot is the opposite of the knotting mode of a slip knot. The difference lies in that the knot state of the hollow knot differs from the knot state of the slip knot after the thread is straightened. After the thread is straightened, the hollow knot is tied firmly and forms a dead knot, while the slip knot is released and disappears. The specific knotting method is not particularly limited or detailed in the present application. Any knotting methods through which the knot is tied firmly and forms a dead knot after the thread is straightened may be used in the present application and are within the scope of the present application.

In the present application, the clamp structure refers to a clamping member which moves in one-way. Particularly, the clamp structure may include a one-way barb structure disposed on the pulling thread and a clamp head interacting with the barb structure to implement one-way locking. After the clamp head and the barb structure are locked, the entire clamp structure can only move backward but cannot move forward. It is to be understood by those skilled in the art that the clamp structure provided in the present application does not rest with its structure or material, but rest with its role and function. Accordingly, anchor structures of any shape that achieves the preceding technical effects may be used in the present application and are within the scope of the present application.

In an embodiment, the cross-sectional area of the small end face of the cam body is less than the cross-sectional area of the large end face of the cam body. **In** the present application, since the cross-sectional area of the small end face of the cam body is less than the cross-sectional area of the large end face of the cam body, the large end face plays a sound engagement effect on the tissue after the cam body enters the skin tissue. The wall thickness of the cam body reduces gradually in the direction from the small end face to the large end face. The large end face with a relatively small wall thickness is easy to engage the tissue tightly, prevent the cam body from falling off, and thus enhance the lifting effect on the skin tissue.

In an embodiment, the pulling thread is a single-stranded thread or a multiple-stranded thread.

In an embodiment, the cam body is a tapered structure.

In an embodiment, the cam body is a solid tapered structure.

In an embodiment, the cam body is formed integrally with the pulling thread. Further in an embodiment, the cam body is formed integrally with the pulling thread by using the hot-pressing injection molding process.

In an embodiment, the cam body is a tapered structure penetrated in the axial direction of the cam body.

In an embodiment, in the direction from the small end face of the cam body to the large end face of the cam body, the wall thickness of the cam body reduces gradually.

In an embodiment, the wall thickness at the small end face of the cam body is in the range of 0.1 mm to 0.9 mm, for example, 0.1 mm, 0.2 mm, 0.3 mm, 0.4 mm, 0.5 mm, 0.6 mm, 0.7 mm, 0.8 mm, or 0.9 mm, and is, for example, 0.3 mm.

In an embodiment, the wall thickness at the large end face of the cam body is in the range of 0.05 mm to 0.2 mm, for example, 0.05 mm, 0.08 mm, 0.1 mm, 0.12 mm, 0.15 mm, 0.18 mm, or 0.2 mm, and is, for example, 0.09 mm.

In an embodiment, the cam body is secured to the pulling thread through an adhesive.

In an embodiment, the cam body is secured to the pulling thread through heat sealing.

In an embodiment, stoppers are disposed between adjacent cam bodies to define the relative positions of the cam bodies. On one hand, the stoppers guarantee the stability of the pulling thread and the cam bodies and avoid relative slippage. On the other hand, after the cam bodies enter the skin tissue, once the positions of the cam bodies are secured, the pulling thread is also defined to a corresponding position so as to perform lifting well.

In an embodiment, the stoppers are thread knots. The stoppers described in the present application are not limited to any structures, forms, or modalities. Any members having the function of position defining may be used in the present application. Moreover, a position-defining mode is that the pulling thread is knotted at spaced intervals to clamp the cam bodies and prevent the cam bodies from slipping on the pulling thread. However, in the present application, the distance between adjacent stoppers is larger than the length of the cam body in the axial direction of the cam body. Accordingly, the cam body may still slip to a small extent between adjacent stoppers but cannot completely slip off from the pulling thread.

In the present application, other engaging reinforcement members may be optionally added to the cam body, enhancing the engagement of the cam body with the skin tissue.

Exemplarily, the engaging reinforcement member is a conical thorn in an oblique cone shape covering the outer surface of the cam body. The tip of the conical thorn faces outward. The bottom surface of the conical thorn contacts the wall of the cam body. The connection line between the tip and the center of the bottom surface has an included angle α against the outer wall surface of the cam body. Optionally, the value range of the included angle satisfies that 10 ° ≤ α ≤ 45 °. In this range, the engaging reinforcement member has a relatively good engagement performance with the tissue. In the case where α < 10 °, the mouth of the engaging reinforcement member is relatively small and is in a closed state after entering the tissue. Even if the one-way lifting embedded thread starts to fall off under force in the reverse direction, the engaging reinforcement member is difficult to open automatically and engage the tissue. **In** the case where α > 45 °, the mouth of the engaging reinforcement member is relatively large. When entering the tissue, the engaging reinforcement member is easy to generate a relatively large resistance and may cause a tissue injury.

Exemplarily, the engaging reinforcement member may a tooth structure disposed at the large end face of the cam body. The arrangement of at least three tooth structures further enhances the engagement strength of the one-way lifting embedded thread with the skin tissue, further improves the engagement performance of the one-way lifting embedded thread with the skin tissue through matching conical thorns, and prevents the cam body from falling off. Accordingly, the one-way lifting embedded thread is firmly and accurately secured inside the skin tissue, and the cam body is prevented from falling off and affecting the lifting on the tissue.

In an embodiment, the cam body and the pulling thread are made of a degradable material.

In an embodiment, the degradable material includes at least one of a synthetic degradable polymer material and a naturally degradable polymer material.

In an embodiment, the synthetic degradable polymer material includes any one or a combination of at least two of polylactic acid, L-polylactic acid, DL-polylactic acid, poly(lactic-co-glycolic acid) copolymer, polycaprolactone, poly(3-hydroxybutyrate-co-3-hydroxyvalerate), polyacetylglutamic acid, polyorthoester, polyethylene oxide/polybutene copolymer, and poly(lactide-co-carpolactone) copolymer.

In an embodiment, the naturally degradable polymer material includes any one or a combination of at least two of collagen, gelatin, chitosan, hyaluronic acid, sodium alginate, and agarose.

In an embodiment, at least one of the surface of the pulling thread and the surface of the cam body is loaded with a drug.

In an embodiment, the drug is selected from one or a combination of at least two of a non-steroid anti-inflammatory drug, a steroid anti-inflammatory drug, an opiate drug, and an analgesic adjuvant.

In a second aspect, the present application provides a lifting device. The lifting device includes a tube-shaped conveyor portion, a handgrip, and the one-way lifting embedded thread described in the first aspect. The tube-shaped conveyor portion includes a secured end and an open end. The secured end is secured to the handgrip.

The lifting end of the pulling thread of the one-way lifting embedded thread is threaded through the inside of the tube-shaped conveyor portion and then out of the open end of the tube-shaped conveyor portion.

In an embodiment, the open end of the tube-shaped conveyor portion is provided with a cam body position adjusting member. The cam body position adjusting member is configured to, when the tube-shaped conveyor portion is withdrawn from the skin tissue, perform at least one of: readjusting the cam body back to a predetermined position, and straightening and tightening the one-way lifting embedded thread.

As described, in the present application, the relative positions of the cam bodies are defined under the position-defining effect of stoppers. Accordingly, the cam bodies cannot slip off but may still slip to a small extent between adjacent stoppers. In this manner, when the tube-shaped conveyor portion is pricked into the skin tissue, the relative positions of the cam bodies inside the skin tissue cannot be guaranteed completely. A possible result is that before the tube-shaped conveyor portion is pricked into the skin tissue, each cam body remains equally spaced apart under the position-defining effect of the stoppers. However, as the tube-shaped conveyor portion is pricked into the skin, under the friction with the pricked hole, the cam bodies slip to a small extend between adjacent stoppers, thus causing offsets in the distance between the cam bodies entering the skin tissue and in the positional relationship of the cam bodies entering the skin tissue. When the free end of the pulling thread is lifted, the skin tissue is forced unevenly under the pulling action of the cam bodies with an irregular positional relationship. Consequently, the lifting effect is affected. Accordingly, in the present application, the cam body position adjusting member is disposed at the open end. In the process of withdrawing the tube-shaped conveyor portion, the one-way lifting embedded thread is secured across the skin tissue by a surgeon and becomes unmovable. Accordingly, as the process of withdrawal progresses, each cam body on the one-way lifting embedded thread passes through the cam body position adjusting member. Under the friction or resistance between the cam body position adjusting member and the cam bodies, the cam bodies return to the positions defined by the stoppers or maintain the same or regular spacing along with the extension of the pulling thread. The cam bodies whose positions are readjusted then enter the skin tissue and become difficult to slip. Consequently, the relative position of each cam body after entering the skin tissue is guaranteed. In an embodiment, the cam body position adjusting member is a clamping baffle disposed at the open end. The clamping baffle is secured inside the tube-shaped conveyor portion. The shape of the clamping baffle is not particularly limited in the present application. Friction or resistance may be applied to the cam body so that the clamping baffle of any structure that makes the cam body returns to the position defined by the stopper may be used in the present application.

In an embodiment, the clamping baffle is secured inside the tube-shaped conveyor portion in the radial direction of the tube-shaped conveyor portion or in the axial direction of the tube-shaped conveyor portion.

In an embodiment, the cam body is elastic. In this manner, after the cam body position adjusting member applies friction or resistance to the cam body, the cam body can smoothly pass through the open end of the tube-shaped conveyor portion, which facilitates the withdrawal of the tube-shaped conveyor portion.

In an embodiment, the clamping baffle is made of a soft plastic material. The soft plastic material may avoid damage when the cam body contacts the clamping baffle so that the subsequent lifting operation is not affected.

In an embodiment, the soft plastic material is any one or a combination of at least two of silicone rubber, polyurethane (PU), polypropylene, high-density polyethylene (HDPE), and polytetrafluoroethylene (PTFE).

In an embodiment, the open end of the tube-shaped conveyor portion is a necking structure recessed inward in the radial direction of the tube-shaped conveyor portion. The necking structure is configured to, when the tube-shaped conveyor portion is withdrawn from the skin tissue, perform at least one of: readjusting the cam body back to the predetermined position, and straightening and tightening the one-way lifting embedded thread.

Similarly, the design concept of the necking structure is similar to the above-mentioned cam body position adjusting member. The expected object is to readjust the positional relationship of the cam body and/or straighten and tighten the one-way lifting embedded thread. **In** the process of withdrawing the tube-shaped conveyor portion from the skin tissue, each cam body passes through the necking structure. Friction is generated between the necking structure and the cam bodies so that the cam bodies are readjusted to the predetermined positions. **In** this manner, the large end faces of the cam bodies are fastened by the stoppers so as to guarantee no offset in the positional relationship of each cam body. Accordingly, it is to be understood by those skilled in the art that other structures or additional added members that may obtain the preceding technical effects may be used in the present application and are within the scope of the present application.

**In** a third aspect, the present application provides a plastic surgery method using the one-way lifting device described in the second aspect to perform lifting. The plastic surgery method includes the steps below.

**In** step (I), the skin tissue is pricked with the lifting device; then the skin tissue is pressed to secure the lifting end of the pulling thread of the one-way lifting embedded thread and the tube-shaped conveyor portion is withdrawn at the same time to make the one-way lifting embedded thread stay in the skin tissue; and then the free end of the pulling thread is lifted to adjust the lifting force to remove wrinkles.

In step (II), the one-way lifting embedded thread is secured with the anchor structure to prevent the one-way lifting embedded thread from completely slipping into the skin tissue.

In an embodiment, before step (I), the plastic surgery method further includes installing the one-way lifting embedded thread.

In an embodiment, the installing includes: tightening the one-way lifting embedded thread, threading the lifting end of the pulling thread through the inside of the tube-shaped conveyor portion and then out of the open end of the tube-shaped conveyor portion by using a threading tool, and then retaining the redundant part of the pulling thread.

In an embodiment, in step (I), the skin tissue is pressed to secure the redundant part of the pulling thread after the threading so as to secure the one-way lifting embedded thread;
In an embodiment, in step (I), while the tube-shaped conveyor portion is being withdrawn from the skin tissue, at least one of the cam body position adjusting member and the necking structure performs at least one of: readjusting the cam body to the predetermined position, and straightening and tightening the one-way lifting embedded thread. Specifically, when the cam body is formed integrally with the pulling thread or is secured to the pulling thread through securing modes including an adhesive or heat sealing, the cam body position adjusting member and/or the necking structure implements the function of straightening and tightening the one-way lifting embedded thread. When the cam body is secured to the pulling thread through stoppers, the cam body may slip to a small extend between adjacent stoppers. **In** this case, the cam body position adjusting member and/or the necking structure may implement the function of readjusting the cam body back to the predetermined position and straightening and tightening the one-way lifting embedded thread.

In an embodiment, the predetermined position is a position where the large end face of the cam body is fastened by the stopper.

In an embodiment, after step (II), the plastic surgery method further includes: securing the one-way lifting embedded thread.

In an embodiment, the securing method includes securing the free end of the pulling thread of the one-way lifting embedded thread to the pricked hole by knotting the free end of the pulling thread of the one-way lifting embedded thread to the skin tissue.

In an embodiment, a plurality of one-way lifting embedded threads are embedded in the same pricked hole by step (I) and step (II); free ends of pulling threads of the one-way lifting embedded threads are knotted in pairs or with each other so as to be secured at the pricked hole.

The numerical range of the present application includes not only the preceding point values but also any point values not listed between the preceding numerical ranges. Due to the limitation of space and for the sake of brevity, the present application does not exhaustively list the specific point values included in the range.

Compared with the related art, the present application has the beneficial effects below.
(1) The present application provides a one-way lifting embedded thread. The arrangement of the anchor structure secures the one-way lifting embedded thread to the skin tissue and prevents the one-way lifting embedded thread from completely slipping into the skin tissue.
(2) The present application further provides a lifting device. The arrangement of the cam body position adjusting member and/or the necking structure that is disposed at the open end of the tube-shaped conveyor portion enables the positions of the cam bodies to be adjusted in the process of withdrawing the needle, guaranteeing no offset in the relative positions when the cam bodies enter the skin tissue. Moreover, this arrangement straightens and tightens the one-way lifting embedded thread so as to prevent the one-way lifting embedded thread from winding inside the skin tissue, causing an uneven force in the process of lifting, and affecting the lifting effect.

### BRIEF DESCRIPTION OF DRAWINGS

FIG. 1 is a view illustrating the structure of a lifting device according to an embodiment of the present application, in which the enlarged region illustrates the position for installing a one-way lifting embedded thread.
FIG. 2 is a view illustrating the structure of a one-way lifting embedded thread according to an embodiment of the present application, in which cam bodies are secured to a pulling thread through stoppers.
FIG. 3 is a view illustrating the structure of a one-way lifting embedded thread according to an embodiment of the present application, in which cam bodies are formed integrally with a pulling thread.
FIG. 4 is a view illustrating the structure of a lifting device (with no one-way lifting embedded thread included) according to an embodiment of the present application, in which the enlarged region illustrates the structure of an open end and a clamping baffle.

### Reference list

- 1: one-way lifting embedded thread
- 11: pulling thread
- 12: cam body
- 2: tube-shaped conveyor portion
- 21: embedded thread outlet
- 22: cam body position adjusting member
- 3: handgrip

### DETAILED DESCRIPTION

Technical solutions of the present application are further described hereinafter through embodiments in conjunction with the drawings.

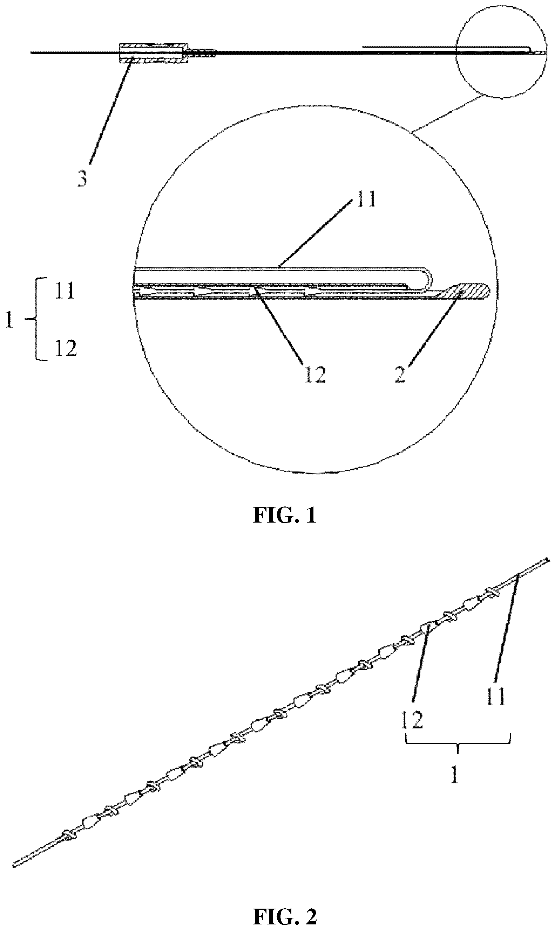

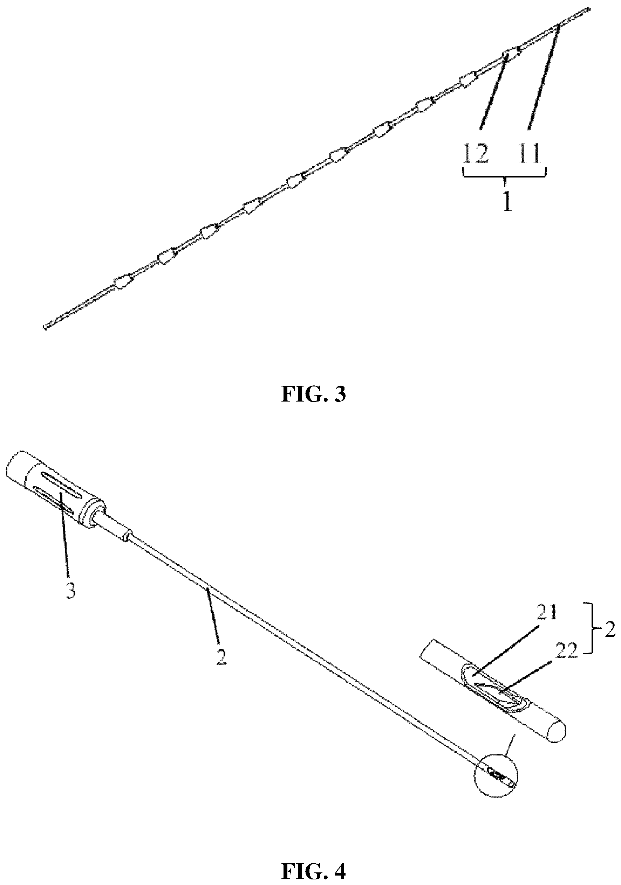

In an embodiment, the present application provides a one-way lifting embedded thread 1. As shown in FIGS. 2 and 3, the one-way lifting embedded thread 1 includes a pulling thread 11 and at least one cam body 12 sleeved on the pulling thread 11. Two ends of the pulling thread 11 are a lifting end and a free end, respectively. The free end is provided with an anchor structure for securing the pulling thread 11. The anchor structure may be selected as a hollow knot or a clamp. Two sides of the cam body 12 are a large end face and a small end face, respectively. The large end face of the cam body 12 faces the free end of the pulling thread 11.

The cam bodies 12 are equally spaced apart or unequally spaced apart in gradient on the pulling thread 11. The distance between two adjacent cam bodies 12 is in the range of 2.5 mm to 15 mm. 4 to 24 cam bodies 12 are sleeved on the pulling thread 11. As for the matching relation between the distance of adjacent cam bodies 12 and the number of cam bodies 12, those skilled in the art may make appropriate adjustments according to the physical condition of a patient and the expected lifting effect of the patient.

The cam body 12 is a tapered structure, optionally including a cone (a truncated cone) and a pyramid (a truncated pyramid). The cam body 12 may be a solid tapered structure or a tapered structure penetrated in the axial direction of the cam body 12.

When the cam body 12 is a solid tapered structure, the securing mode of the cam body 12 to the pulling thread 11 is shown in FIG. 3. The one-way lifting embedded thread 1 is formed integrally using the hot-pressing injection molding process. When the cam body 12 is a tapered structure penetrated in the axial direction of the cam body 12, in the direction from the small end face to the large end face, the wall thickness of the cam body 12 reduces gradually. Optionally, the cam body 12 may be secured to the pulling thread 11 through an adhesive, heat sealing, or stoppers. In an embodiment, as shown in FIG. 2, the positions of the cam bodies 12 are defined by knotting the pulling thread.

The cam body 12 and the pulling thread 11 are made of a degradable material that does not need to be taken out after the surgery but is degraded in the body with no toxic or side effects on the human body. The degradable material includes at least one of a synthetic degradable polymer material and a naturally degradable polymer material. The synthetic degradable polymer material includes any one or a combination of at least two of polylactic acid, L-polylactic acid, DL-polylactic acid, poly(lactic-co-glycolic acid) copolymer, polycaprolactone, poly(3-hydroxybutyrate-co-3-hydroxyvalerate), polyacetylglutamic acid, polyorthoester, polyethylene oxide/polybutene copolymer, and poly(lactide-co-carpolactone) copolymer. The naturally degradable polymer material includes any one or a combination of at least two of collagen, gelatin, chitosan, hyaluronic acid, sodium alginate, and agarose.

At least one of the surface of the pulling thread 11 and the surface of the cam body 12 is loaded with a drug. The drug is selected from one or a combination of at least two of a non-steroid anti-inflammatory drug, a steroid anti-inflammatory drug, an opiate drug, and an analgesic adjuvant. The drug may be loaded on the surface of the pulling thread 11 or the surface of the cam body 12. Alternatively, the drug may be loaded on the surface of the pulling thread 11 and the surface of the cam body 12.

In another embodiment, the present application provides a lifting device as shown in FIG. 1. The lifting device includes a tube-shaped conveyor portion 2, a handgrip 3, and the one-way lifting embedded thread 1 described in the preceding embodiment. The tube-shaped conveyor portion 2 includes a secured end and an open end. The secured end is secured to the handgrip 3. The open end is provided with an embedded thread outlet 21. (For the specific structure, refer to the partially enlarged view in FIG. 1. The partially enlarged region illustrates the structure of the one-way lifting embedded thread 1 and the state when one-way lifting embedded thread 1 is threaded out of the embedded thread outlet 21.)

The lifting end of the pulling thread 11 of the one-way lifting embedded thread 1 is threaded through the inside of the tube-shaped conveyor portion 2 and then out of the embedded thread outlet 21. Terms "thread out of" and "thread through" herein refer to positional relationships and states during installation, and do not refer to installation actions or methods.

The embedded thread outlet 21 is provided with a cam body position adjusting member 22. The cam body position adjusting member 22 is configured to readjust the cam body 12 to back predetermined positions when the tube-shaped conveyance portion 2 is withdrawn from the skin tissue. Specifically, an optional cam body position adjusting member 22 is a clamping baffle disposed at the embedded thread outlet 21. (For the specific structure and positional relationship of the clamping baffle, refer to the partially enlarged view in FIG. 4.) The clamping baffle is made of a soft plastic material. Optionally, the soft plastic material is any one or a combination of at least two of silicone rubber, polyurethane (PU), polypropylene (PP), high-density polyethylene (HDPE), and polytetrafluoroethylene (PTFE).

Additionally, if the cam body position adjusting member 22 is not provided, a necking structure recessed inward in the radial direction of the tube-shaped conveyor portion 2 may be designed at the open end of the tube-shaped conveyor portion 2. The necking structure is configured to readjust the cam body 12 back to predetermined position when the tube-shaped conveyor portion 2 is withdrawn from the skin tissue.

It is to be understood by those skilled in the art that a simultaneous provision of the optional cam body position adjusting member 22 and the necking structure is not limited in the present application.

In another embodiment, the present application provides a plastic surgery method using the preceding lifting device to perform lifting. The plastic surgery method includes the steps below.

Threading is implemented before the surgery. Specifically, the threading process includes: tightening the one-way lifting embedded thread 1; threading the lifting end of the pulling thread 11 into the handgrip 3 of the lifting device, through the inside of the tube-shaped conveyor portion 2, and then out of the embedded thread outlet 21 by using a threading tool; retaining the redundant part of the pulling thread 11; and then bending the redundant part of the pulling thread 11. The redundant part of the pulling thread 11 is used for facilitating the surgeon in pressing the skin tissue to securing the one-way lifting embedded thread 1 when withdrawing the needle in the surgery. Accordingly, the one-way lifting embedded thread 1 is prevented from being withdrawn along with the tube-shaped conveyor portion 2 while the needle is being withdrawn. Terms "thread into" and "thread out of" herein refer to installation actions or methods, and do not refer to installation states and positional relationships.

The surgery process includes the steps below.

In step (I), the skin tissue is pricked with the lifting device. As for the cam body 12 on the one-way lifting embedded thread 1, the cross-sectional area increases gradually from the small end face to the large end face, which facilitates entering the tissue and mitigating an injury to the tissue. Then the skin tissue is pressed to secure the lifting end of the pulling thread 11 of the one-way lifting embedded thread 1 (that is, the redundant part of the pulling thread 11 retained in threading before the surgery) and the tube-shaped conveyor portion 2 is withdrawn slowly at the same time.

In step (II), at the same time of when the tube-shaped conveyor portion 2 is withdrawn, the skin tissue is pressed by the surgeon to secure the lifting end of the pulling thread 11 to become unmovable. In this case, the tube-shaped conveyor portion 2 is withdrawn while the one-way lifting embedded thread 1 still stays in the skin tissue. While the tube-shaped conveyor portion 2 is being withdrawn from the skin tissue, the cam body position adjusting member 22 and/or the necking structure that is disposed at the embedded thread outlet 21 readjusts the cam bodies 12 to the positions of the stoppers and/or straightens and tightens the one-way lifting embedded thread 1 so that large end faces of the cam bodies 12 are fastened by the stoppers, guaranteeing no offset in the relative position of each cam body 12 entering the skin tissue. In this case, the skin tissue is forced evenly under the lifting action of the cam bodies 12.

In step (III), after the tube-shaped conveyor portion 2 is withdrawn, most of the one-way lifting embedded thread 1 stays in the skin tissue. However, the free end of the pulling thread 11 of the one-way lifting embedded thread 1 stays outside the skin tissue. The surgeon lifts the free end of the pulling thread 11 to adjust the lifting force to remove wrinkles.

After the surgery, the free end of the pulling thread 11 is secured to the pricked hole by knotting the free end of the pulling thread 11 to the skin tissue. The one-way lifting embedded thread 1 stays in the skin tissue. The one-way lifting embedded thread 1 is made of a biodegradable material that is degradable inside the human tissue, thus causing no toxic or side effects on the human body.

In the process of surgery, a plurality of one-way lifting embedded threads 1 are embedded in the same pricked hole by repeating step (I) and step (II). After embedding, free ends of pulling threads 11 of the one-way lifting embedded threads 1 are knotted in pairs or with each other so as to be secured at the pricked hole. This reduces the number of pricked holes, implements lifting on multiple parts, and avoids apparent surgical signs or irretrievable scars after the surgery.

### Embodiment one

This embodiment provides a one-way lifting embedded thread 1. The one-way lifting embedded thread 1 includes a pulling thread 11 and four cam bodies 12 sleeved on the pulling thread 11. Each cam body 12 is equally spaced apart on the pulling thread 11. The distance between two adjacent cam bodies 12 is 15 mm. Thread knots are formed between adjacent cam bodies 12 as stoppers by knotting the pulling thread 11 to define relative positions of the adjacent cam bodies 12.

Two ends of the pulling thread 11 are a lifting end and a free end, respectively. The free end is provided with a hollow knot for securing the pulling thread. The hollow knot at the free end replaces part of the cam bodies in the two-way lifting embedded thread that are configured to clamp the skin tissue, thus preventing the one-way lifting embedded thread 1 from completely slipping into the skin tissue, enhancing the lifting force, and improving lifting duration.

Two sides of a cam body 12 are a large end face and a small end face, respectively. The large end face faces the free end of the pulling thread 11. Naturally, the small end face faces the lifting end of the pulling thread 11. The cam body 12 is a tapered structure penetrated in the axial direction of the cam body 12. The wall thickness at the small end face is 0.9 mm. The wall thickness at the large end face is 0.2 mm. In the direction from the small end face to the large end face, the wall thickness of the cam body 12 reduces gradually. That is, the wall thickness of the cam body 12 reduces evenly from 0.9 mm to 0.2 mm.

The cam bodies 12 and the pulling thread 11 are made of a degradable material that does not need to be taken out after the surgery but is degraded in the body. The surface of the cam bodies 12 and the surface of the pulling thread 11 are loaded with a non-steroid anti-inflammatory drug.

This embodiment further provides a lifting device. The lifting device includes a tube-shaped conveyor portion 2, a handgrip 3, and the preceding one-way lifting embedded thread 1. The tube-shaped conveyor portion 2 includes a secured end and an open end. The secured end is secured to the handgrip 3. The open end is provided with an embedded thread outlet 21. The lifting end of the pulling thread 11 of the one-way lifting embedded thread 1 is threaded through the inside of the tube-shaped conveyor portion 2 and then out of the embedded thread outlet 21.

A clamping baffle made of silicone rubber is disposed at the embedded thread outlet 21. The clamping baffle is configured to, when the tube-shaped conveyor portion 2 is withdrawn from the skin tissue, readjust the cam bodies 12 back to predetermined positions and straighten and tighten the one-way lifting embedded thread 1. The clamping baffle is secured inside the tube-shaped conveyor portion 2 in the radial direction of the tube-shaped conveyor portion 2.

In the process of using the preceding lifting device to implement the plastic surgery to remove wrinkles, as the tube-shaped conveyor portion 2 is withdrawn from the skin tissue, the clamping baffle disposed at the embedded thread outlet 21 applies friction to the cam bodies 12. On one hand, the cam bodies 12 are readjusted to the positions of the thread knots so that large end faces of the cam bodies 12 are fastened by the thread knots, guaranteeing no offset in the relative position of each cam body 12 entering the skin tissue. In this case, the skin tissue is forced evenly under the lifting action of the cam bodies 12. On the other hand, friction applied to the cam bodies 12 helps straighten and tighten the one-way lifting embedded thread 1 so as to prevent the one-way lifting embedded thread 1 from winding inside the skin tissue, causing an uneven force in the process of lifting, and affecting the lifting effect.

### Embodiment two

This embodiment provides a one-way lifting embedded thread 1. The one-way lifting embedded thread 1 includes a pulling thread 11 and fourteen cam bodies 12 sleeved on the pulling thread 11. Each cam body 12 is equally spaced apart on the pulling thread 11. The distance between two adjacent cam bodies 12 is 5.5 mm. The cam bodies are secured to the pulling thread 11 through an adhesive.

Two ends of the pulling thread 11 are a lifting end and a free end, respectively. The free end is provided with a clamp for securing the pulling thread 11. Specifically, the clamp structure may include a one-way barb structure disposed on the pulling thread 11 and a clamp head interacting with the barb structure to implement one-way locking. After the clamp head and the barb structure are locked, the entire clamp structure can only move backward but cannot move forward. The clamp structure replaces part of the cam bodies in the two-way lifting embedded thread that are configured to clamp the skin tissue, thus preventing the one-way lifting embedded thread 1 from completely slipping into the skin tissue, enhancing the lifting force, and improving lifting duration.

Two sides of a cam body 12 are a large end face and a small end face. The large end face faces the free end of the pulling thread 11. The small end face faces the lifting end of the pulling thread 11. The cam body 12 is a tapered structure penetrated in the axial direction of the cam body 12. The wall thickness at the small end face is 0.1 mm. The wall thickness at the large end face is 0.05 mm. In the direction from the small end face to the large end face, the wall thickness of the cam body 12 reduces gradually. That is, the wall thickness of the cam body 12 reduces evenly from 0.1 mm to 0.05 mm.

The cam bodies 12 and the pulling thread 11 are made of a degradable material that does not need to be taken out after the surgery but is degraded in the body. The surface of the cam bodies 12 and the surface of the pulling thread 11 are loaded with a steroid anti-inflammatory drug.

This embodiment further provides a lifting device. The lifting device includes a tube-shaped conveyor portion 2, a handgrip 3, and the preceding one-way lifting embedded thread 1. The tube-shaped conveyor portion 2 includes a secured end and an open end. The secured end is secured to the handgrip 3. The open end is provided with an embedded thread outlet 21. The lifting end of the pulling thread 11 of the one-way lifting embedded thread 1 is threaded through the inside of the tube-shaped conveyor portion 2 and then out of the embedded thread outlet 21.

A necking structure recessed inward in the radial direction of the tube-shaped conveyor portion 2 is designed at the open end of the tube-shaped conveyor portion 2. The necking structure is configured to straighten and tighten the one-way lifting embedded thread 1 when the tube-shaped conveyor portion 2 is withdrawn from the skin tissue so as to prevent the one-way lifting embedded thread 1 from winding inside the skin tissue.

In the process of using the preceding lifting device to implement the plastic surgery to remove wrinkles, as the tube-shaped conveyor portion 2 is withdrawn from the skin tissue, the necking structure applies friction to the cam bodies 12 and thus straightens and tightens the one-way lifting embedded thread 1 so as to prevent the one-way lifting embedded thread 1 from winding inside the skin tissue, causing an uneven force in the process of lifting, and affecting the lifting effect.

### Embodiment three

This embodiment provides a one-way lifting embedded thread 1. The one-way lifting embedded thread 1 includes a pulling thread 11 and ten cam bodies 12 sleeved on the pulling thread 11. Each cam body 12 is equally spaced apart on the pulling thread 11. The distance between two adjacent cam bodies 12 is 5.5 mm. The cam bodies are secured to the pulling thread 11 through heat sealing.

Two ends of the pulling thread 11 are a lifting end and a free end, respectively. The free end is provided with a hollow knot for securing the pulling thread 11. The hollow knot replaces part of the cam bodies in the two-way lifting embedded thread that are configured to clamp the skin tissue, thus preventing the one-way lifting embedded thread 1 from completely slipping into the skin tissue, enhancing the lifting force, and improving lifting duration.

Two sides of a cam body 12 are a large end face and a small end face. The large end face faces the free end of the pulling thread 11. The small end face faces the lifting end of the pulling thread 11. The cam body 12 is a tapered structure penetrated in the axial direction of the cam body 12. The wall thickness at the small end face is 0.3 mm. The wall thickness at the large end face is 0.09 mm. In the direction from the small end face to the large end face, the wall thickness of the cam body 12 reduces gradually. That is, the wall thickness of the cam body 12 reduces evenly from 0.3 mm to 0.09 mm.

The cam bodies 12 and the pulling thread 11 are made of a degradable material that does not need to be taken out after the surgery but is degraded in the body. The surface of the cam bodies 12 and the surface of the pulling thread 11 are loaded with an opiate drug.

This embodiment further provides a lifting device. The lifting device includes a tube-shaped conveyor portion 2, a handgrip 3, and the preceding one-way lifting embedded thread 1. The tube-shaped conveyor portion 2 includes a secured end and an open end. The secured end is secured to the handgrip 3. The open end is provided with an embedded thread outlet 21. The lifting end of the pulling thread 11 of the one-way lifting embedded thread 1 is threaded through the inside of the tube-shaped conveyor portion 2 and then out of the embedded thread outlet 21.

A clamping baffle made of HDPE is disposed at the embedded thread outlet 21. Moreover, a necking structure recessed inward in the radial direction of the tube-shaped conveyor portion 2 is designed at the open end of the tube-shaped conveyor portion 2. The clamping baffle and the necking structure are configured to straighten and tighten the one-way lifting embedded thread when the tube-shaped conveyor portion 2 is withdrawn from the skin tissue so as to prevent the one-way lifting embedded thread 1 from winding inside the skin tissue. The clamping baffle is secured inside the tube-shaped conveyor portion 2 in the axial direction of tube-shaped conveyor portion 2.

In the process of using the preceding lifting device to implement the plastic surgery to remove wrinkles, as the tube-shaped conveyor portion 2 is withdrawn from the skin tissue, the clamping baffle disposed at the embedded thread outlet 21 and the necking structure designed at the open end apply friction to the cam bodies 12 and thus straightens and tightens the one-way lifting embedded thread 1 so as to prevent the one-way lifting embedded thread 1 from winding inside the skin tissue, causing an uneven force in the process of lifting, and affecting the lifting effect.

As declared by the applicant, the preceding are only embodiments of the present application and are not intended to limit the scope of the present application. Apparently, those skilled in the art should know that it is easy for those skilled in the art to conceive modifications or substitutions within the scope of the present application. These modifications or substitutions are within the scope of the present application.

## Claims

1. A lifting device, **characterized by** a tube-shaped conveyor portion (2), a handgrip (3), and a one-way lifting embedded thread, wherein the one-way lifting embedded thread comprises a pulling thread (11) and at least one cam body (12) sleeved on the pulling thread (11),
wherein two ends of the pulling thread are a lifting end and a free end, respectively, and the free end is provided with an anchor structure for securing the pulling thread and preventing the pulling thread (11) from moving toward the lifting end, the free end is configured to stay outside a skin tissue through the anchor structure and allow adjustment of a lifting force of the pulling thread (11) after the pulling thread (11) is threaded into the skin issue, and the lifting end is configured to stay inside the skin tissue after the free end is secured by the anchor structure for one-way lifting; and
two sides of one of the at least one cam body (12) are a large end face and a small end face, respectively, and the large end face of the one of the at least one cam body (12) faces the free end of the pulling thread (11);
wherein the anchor structure comprises a clamp structure, the clamp structure comprises a one-way barb structure disposed on the pulling thread (11) and a clamp head interacting with the barb structure to implement one-way locking, and the clamp structure is configured to be capable of moving only towards the free end after the clamp head and the barb structure are locked;
wherein the tube-shaped conveyor portion (2) comprises a secured end and an open end, and the secured end is secured to the handgrip (3), and
the lifting end of the pulling thread (11) of the one-way lifting embedded thread is threaded through an inside of the tube-shaped conveyor portion (2) to and then out of the open end of the tube-shaped conveyor portion (2);
wherein the open end of the tube-shaped conveyor portion (2) is provided with a cam body position adjusting member (22), and the cam body position adjusting member (22) is configured to, when the tube-shaped conveyor portion (2) is withdrawn from skin tissue, readjust the at least one cam body (12) back to a predetermined position, and straighten and tighten the one-way lifting embedded thread; and
wherein the cam body position adjusting member (22) is a clamping baffle disposed at the open end of the tube-shaped conveyor portion (2), and the clamping baffle is secured inside the tube-shaped conveyor portion (2).

2. The lifting device according to claim 1, wherein the open end of the tube-shaped conveyor portion (2) is a necking structure recessed inward in a radial direction of the tube-shaped conveyor portion (2), and the necking structure is configured to, when the tube-shaped conveyor portion (2) is withdrawn from skin tissue, readjust the at least one cam body (12) back to a predetermined position, and straighten and tightening the one-way lifting embedded thread.

3. The lifting device according to claim 1, wherein 4 to 24 cam bodies (12) are sleeved on the pulling thread (11).

4. The lifting device according to claim 3, wherein the cam bodies (12) are equally spaced apart on the pulling thread (11), or the cam bodies (12) are unequally spaced apart in gradient on the pulling thread (11).

5. The lifting device according to any one of claims 1, 3 and 4, wherein the one of the at least one cam body (12) is a tapered structure.

6. The lifting device according to any one of claims 1, 3 and 4, wherein the at least one cam body (12) is disposed on the pulling thread (11) in at least one of the following manners:
the at least one cam body (12) is formed integrally with the pulling thread (11);
the at least one cam body (12) is secured to the pulling thread (11) through an adhesive;
the at least one cam body (12) is secured to the pulling thread (11) through heat sealing; and
a stopper is disposed between two adjacent ones of the at least one cam body (12) to define a relative position of the at least one cam body (12).

7. The lifting device according to any one of claims 1, 3 and 4, wherein the one of the at least one cam body (12) is a solid tapered structure, or a tapered structure penetrated in an axial direction of the one of the at least one cam body (12).

8. The lifting device according to any one of claims 1, 3 and 4, wherein the at least one cam body (12) and the pulling thread (11) are made of a degradable material.

9. The lifting device according to any one of claims 1, 3 and 4, wherein at least one of a surface of the pulling thread (11) and a surface of the at least one cam body (12) is loaded with a drug.

## Patentansprüche

1. Hebevorrichtung, **gekennzeichnet durch** einen rohrförmigen Förderabschnitt (2), einen Handgriff (3) und einen Einweg-Hebeeinbettungsfaden, wobei der Einweg-Hebeeinbettungsfaden einen Zugfaden (11) und mindestens einen auf den Zugfaden (11) aufgeschobenen Nockenkörper (12) umfasst,
wobei die beiden Enden des Zugfadens jeweils ein Hebeende und ein freies Ende sind und das freie Ende mit einer Verankerungsstruktur versehen ist, um den Zugfaden zu sichern und zu verhindern, dass sich der Zugfaden (11) in Richtung des Hebeendes bewegt, wobei das freie Ende so konfiguriert ist, dass es durch die Verankerungsstruktur außerhalb eines Hautgewebes verbleibt und eine Einstellung der Hebekraft des Zugfadens (11) ermöglicht, nachdem der Zugfaden (11) in das Hautgewebe eingefädelt wurde, während das Hebeende so konfiguriert ist, dass es innerhalb des Hautgewebes verbleibt, nachdem das freie Ende durch die Verankerungsstruktur für ein Einweg-Anheben gesichert wurde; und
wobei zwei Seiten eines des mindestens einen Nockenkörpers (12) jeweils eine große Endfläche und eine kleine Endfläche sind, wobei die große Endfläche des einen des mindestens einen Nockenkörpers (12) dem freien Ende des Zugfadens (11) zugewandt ist;
wobei die Verankerungsstruktur eine Klemmstruktur umfasst, die eine Einweg-Widerhakenstruktur, die auf dem Zugfaden (11) angeordnet ist, und einen Klemmkopf umfasst, der mit der Widerhakenstruktur zusammenwirkt, um eine Einwegverriegelung zu bewirken, wobei die Klemmstruktur so konfiguriert ist, dass sie sich nur in Richtung des freien Endes bewegen kann, nachdem der Klemmkopf und die Widerhakenstruktur verriegelt wurden;
wobei der rohrförmige Förderabschnitt (2) ein gesichertes Ende und ein offenes Ende umfasst und das gesicherte Ende an dem Handgriff (3) gesichert ist, und
wobei das Hebeende des Zugfadens (11) des Einweg-Hebeeinbettungsfadens durch ein Inneres des rohrförmigen Förderabschnitts (2) zu dem offenen Ende des rohrförmigen Förderabschnitts (2) und dann aus diesem heraus gefädelt ist;
wobei das offene Ende des rohrförmigen Förderabschnitts (2) mit einem Nockenkörperpositions-Einstellelement (22) versehen ist und das Nockenkörperpositions-Einstellelement (22) so konfiguriert ist, dass es, wenn der rohrförmige Förderabschnitt (2) aus dem Hautgewebe zurückgezogen wird, den mindestens einen Nockenkörper (12) wieder in eine vorbestimmte Position zurückstellt und den Einweg-Hebeeinbettungsfaden begradigt und strafft; und
wobei das Nockenkörperpositions-Einstellelement (22) eine Klemmblende ist, die am offenen Ende des rohrförmigen Förderabschnitts (2) angeordnet ist, wobei die Klemmblende innerhalb des rohrförmigen Förderabschnitts (2) gesichert ist.

2. Hebevorrichtung nach Anspruch 1, wobei das offene Ende des rohrförmigen Förderabschnitts (2) eine in radialer Richtung des rohrförmigen Förderabschnitts (2) nach innen vertiefte Einschnürungsstruktur ist, wobei die Einschnürungsstruktur so konfiguriert ist, dass sie, wenn der rohrförmige Förderabschnitt (2) aus dem Hautgewebe zurückgezogen wird, den mindestens einen Nockenkörper (12) wieder in eine vorbestimmte Position zurückstellt und den Einweg-Hebeeinbettungsfaden begradigt und strafft.

3. Hebevorrichtung nach Anspruch 1, wobei 4 bis 24 Nockenkörper (12) auf den Zugfaden (11) aufgeschoben sind.

4. Hebevorrichtung nach Anspruch 3, wobei die Nockenkörper (12) in gleichen Abständen auf dem Zugfaden (11) angeordnet sind oder die Nockenkörper (12) in ungleichen Abständen in einem Gradienten auf dem Zugfaden (11) angeordnet sind.

5. Hebevorrichtung nach einem der Ansprüche 1, 3 und 4, wobei der eine des mindestens einen Nockenkörpers (12) eine konische Struktur ist.

6. Hebevorrichtung nach einem der Ansprüche 1, 3 und 4, wobei der mindestens eine Nockenkörper (12) auf mindestens eine der folgenden Weisen auf dem Zugfaden (11) angeordnet ist:
der mindestens eine Nockenkörper (12) ist integral mit dem Zugfaden (11) ausgebildet;
der mindestens eine Nockenkörper (12) ist durch einen Klebstoff an dem Zugfaden (11) gesichert;
der mindestens eine Nockenkörper (12) ist durch Heißsiegeln an dem Zugfaden (11) gesichert; und
ein Anschlag ist zwischen zwei benachbarten des mindestens einen Nockenkörpers (12) angeordnet, um eine relative Position des mindestens einen Nockenkörpers (12) zu definieren.

7. Hebevorrichtung nach einem der Ansprüche 1, 3 und 4, wobei der eine des mindestens einen Nockenkörpers (12) eine massive konische Struktur oder eine konische Struktur ist, die in axialer Richtung des einen des mindestens einen Nockenkörpers (12) durchdrungen ist.

8. Hebevorrichtung nach einem der Ansprüche 1, 3 und 4, wobei der mindestens eine Nockenkörper (12) und der Zugfaden (11) aus einem abbaubaren Material bestehen.

9. Hebevorrichtung nach einem der Ansprüche 1, 3 und 4, wobei mindestens eine von einer Oberfläche des Zugfadens (11) und einer Oberfläche des mindestens einen Nockenkörpers (12) mit einem Medikament beschichtet ist.

## Revendications

1. Dispositif de lifting, **caractérisé par** une partie convoyeuse en forme de tube (2), une poignée (3), et un fil de lifting intégré à sens unique, dans lequel le fil de lifting intégré à sens unique comprend un fil de traction (11) et au moins un corps de came (12) emmanché sur le fil de traction (11),
dans lequel deux terminaisons du fil de traction sont un terminaison de lifting et une terminaison libre, respectivement, et la terminaison libre est munie d'une structure d'ancrage pour sécuriser le fil de traction et empêcher le fil de traction (11) de se déplacer vers la terminaison de lifting, et la terminaison libre est configurée pour rester à l'extérieur d'un tissu cutané via la structure d'ancrage et permettre l'ajustement de la force de lifting du fil de traction (11) une fois que le fil de traction (11) est enfilé dans le tissu cutané, et la terminaison de lifting est configurée pour rester à l'intérieur du tissu cutané une fois que la terminaison libre est sécurisée par la structure d'ancrage pour lifting à sens unique ; et
deux côtés de l'un des au moins un corps de came (12) sont une grande face terminale et une petite face terminale, respectivement, et la grande face terminale de l'un des au moins un corps de came (12) fait face à la terminaison libre du fil de traction (11) ;
dans lequel la structure d'ancrage comprend une structure de serrage, la structure de serrage comprend une structure à aspérités à sens unique agencée sur le fil de traction (11) et une tête de serrage interagissant avec la structure à aspérités pour mettre en œuvre un verrouillage à sens unique, et la structure de serrage est configurée pour être capable de se déplacer uniquement vers la terminaison libre une fois que la tête de serrage et la structure à aspérités sont verrouillées ;
dans lequel la partie convoyeuse en forme de tube (2) comprend une terminaison sécurisée et un terminaison ouverte, et la terminaison sécurisée est sécurisée à la poignée (3), et
la terminaison de lifting du fil de traction (11) du fil de lifting intégré à sens unique est enfilée à travers l'intérieur de la partie convoyeuse en forme de tube (2) jusqu'à, puis à l'extérieur de, la terminaison ouverte de la partie convoyeuse en forme de tube (2) ;
dans lequel la terminaison ouverte de la partie convoyeuse en forme de tube (2) est munie d'un élément d'ajustement de position du corps de came (22), et l'élément d'ajustement de position du corps de came (22) est configuré pour, lorsque la partie convoyeuse en forme de tube (2) est retirée du tissu cutané, réajuster le au moins un corps de came (12) sur une position prédéterminée, et redresser et serrer le fil intégré de lifting à sens unique ; et
dans lequel l'élément d'ajustement de position du corps de came (22) est un déflecteur de serrage agencé à la terminaison ouverte de la partie convoyeuse en forme de tube (2), et le déflecteur de serrage est sécurisé à l'intérieur de la partie convoyeuse en forme de tube (2).

2. Dispositif de lifting selon la revendication 1, dans lequel la terminaison ouverte de la partie convoyeuse en forme de tube (2) est une structure d'étranglement renfoncée vers l'intérieur dans le sens radial de la partie convoyeuse en forme de tube (2), et la structure d'étranglement est configurée pour, lorsque la partie convoyeuse en forme de tube (2) est retirée du tissu cutané, réajuster le au moins un corps de came (12) sur une position prédéterminée, et redresser et serrer le fil intégré de lifting à sens unique.

3. Dispositif de lifting selon la revendication 1, dans lequel 4 à 24 corps de cames (12) sont emmanchées sur le fil de traction (11).

4. Dispositif de lifting selon la revendication 3, dans lequel les corps de came (12) sont espacés de façon égale sur le fil de traction (11), ou les corps de came (12) sont espacés de façon inégale en gradients sur le fil de traction (11).

5. Dispositif de lifting selon l'une quelconque des revendications 1, 3 et 4, dans lequel l'un des au moins un corps de came (12) est une structure conique.

6. Dispositif de lifting selon l'une quelconque des revendications 1, 3 et 4, dans lequel le au moins un corps de came (12) est agencé sur le fil de traction (11) selon au moins une des manières suivantes :
le au moins un corps de came (12) est formé intégralement avec le fil de traction (11) ;
le au moins un corps de came (12) est sécurisé au fil de traction (11) via un adhésif ;
le au moins un corps de came (12) est sécurisé au fil de traction (11) via thermoscellage ; et
un bouchon est agencé entre deux des au moins un corps de came (12) adjacents pour définir une position relative du au moins un corps de came (12).

7. Dispositif de lifting selon l'une quelconque des revendications 1, 3 et 4, dans lequel l'un des au moins un corps de came (12) est une structure conique, ou une structure conique pénétrée dans le sens axial de l'un des au moins un corps de came (12).

8. Dispositif de lifting selon l'une quelconque des revendications 1, 3 et 4, dans lequel le au moins un corps de came (12) et le fil de traction (11) sont en matériau dégradable.

9. Dispositif de lifting selon l'une quelconque des revendications 1, 3 et 4, dans lequel au moins l'une parmi une surface du fil de traction (11) et une surface du au moins un corps de came (12) contient un médicament.
